Europäisches Patentamt

European Patent Office

Office européen des brevets

(19)

(11) **EP 1 649 803 A1**

(12) **EUROPEAN PATENT APPLICATION**

(43) Date of publication:
**26.04.2006 Bulletin 2006/17**

(51) Int Cl.:
**A61B 5/00** (2006.01)

(21) Application number: **05003466.9**

(22) Date of filing: **17.02.2005**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **19.10.2004 JP 2004304799**

(71) Applicant: **Hitachi Ltd.**
**Tokyo 100-8280 (JP)**

(72) Inventors:
• **Cho, Ok-Kyung, c/o Hitachi Ltd., IPD.**
**Chiyoda-ku,**
**Tokyo 100-8220 (JP)**

• **Kim, Yoon-Ok, c/o Hitachi Ltd., IPD.**
**Chiyoda-ku,**
**Tokyo 100-8220 (JP)**
• **Nagata, Koji, c/o Hitachi Ltd., IPD.**
**Chiyoda-ku,**
**Tokyo 100-8220 (JP)**
• **Mitsumaki, Hiroshi, c/o Hitachi Ltd., IPD.**
**Chiyoda-ku,**
**Tokyo 100-8220 (JP)**

(74) Representative: **Strehl Schübel-Hopf & Partner**
**Maximilianstrasse 54**
**80538 München (DE)**

(54) **Blood sugar level measuring apparatus**

(57) Blood sugar levels are measured non-invasively based on temperature measurement. Measurement data is stabilized by correcting a non-invasive blood sugar level measurement value obtained by a temperature measuring system on the basis of the blood oxygen saturation and the blood flow volume. A heat conducting member is provided in a blood flow volume measurement portion that measures the blood flow volume based on temperature detection. The heat conducting member is made up of a cylindrical main body 61 and a plate-like support plate 63 disposed in the longitudinal direction of an internal cavity of the main body. A first temperature detector 23 is fixed to an end portion of the support plate and is disposed within the internal cavity of the main body. A second temperature detector 24 is fixed on an external surface of the main body.

FIG. 11

**Description**

CLAIM OF PRIORITY

The present application claims priority from Japanese application JP 2004-304799 filed on October 19, 2004, the content of which is hereby incorporated by reference into this application.

CROSS REFERENCE TO RELATED APPLICATION

US Patent application No. 10/620,689 is a co-pending application of this application. The content of which is incorporated herein by cross-reference.

BACKGROUND OF THE INVENTION

Field of the Invention

The present invention relates to a method and apparatus for non-invasive measurement of blood sugar levels for measuring glucose concentration in a living body without blood sampling.

Description of Related Art

Hilson *et al.* report facial and sublingual temperature changes in diabetics following intravenous glucose injection (Non-Patent Document 1). Scott *et al.* discuss the issue of diabetics and thermoregulation (Non-Patent Document 2). Based on the knowledge gained from such researches, Cho *et al.* suggest a method and apparatus for determining blood glucose concentration by temperature measurement without requiring the collection of a blood sample (Patent Documents 1 and 2).
Various other attempts have been made to determine glucose concentration without blood sampling. For example, a method has been suggested (Patent Document 3) whereby a measurement site is irradiated with near-infrared light of three wavelengths, and the intensity of transmitted light as well as the temperature of the living body is detected. A representative value of the second-order differentiated value of absorbance is then calculated, and the representative value is corrected in accordance with the difference between the living body temperature and a predetermined reference temperature. The blood sugar concentration corresponding to the thus corrected representative value is then determined. An apparatus is also provided (Patent Document 4) whereby a measurement site is heated or cooled while monitoring the living body temperature. The degree of attenuation of light based on light irradiation is measured at the moment of temperature change so that the glucose concentration responsible for the temperature-dependency of the degree of light attenuation can be measured. Further, an apparatus is reported (Patent Document 5) whereby an output ratio between reference light and transmitted light following the irradiation of the sample is taken, and then a glucose concentration is calculated in accordance with a linear expression of the logarithm of the output ratio and the living body temperature.
[Non-Patent Document 1] Diabete & Metabolisme, "Facial and sublingual temperature changes following intravenous glucose injection in diabetics" by R.M. Hilson and T.D.R. Hockaday, 1982, 8, 15-19
[Non-Patent Document 2] Can. J. Physiol. Pharmacol., "Diabetes mellitus and thermoregulation" by A.R. Scott, T. Bennett, I.A. MacDonald, 1987, 65, 1365-1376
[Patent Document 1] U.S. Patent No. 5,924,996
[Patent Document 2] U.S. Patent No. 5,795,305
[Patent Document 3] JP Patent Publication (Kokai) No. 2000-258343 A
[Patent Document 4] JP Patent Publication (Kokai) No. 10-33512 A (1998)
[Patent Document 5] JP Patent Publication (Kokai) No. 10-108857 A (1998)

SUMMARY OF THE INVENTION

Glucose (blood sugar) in blood is used for glucose oxidation reaction in cells to produce necessary energy for the maintenance of living bodies. In the basal metabolism state, in particular, most of the produced energy is converted into heat energy for the maintenance of body temperature. Thus, it can be expected that there is some relationship between blood glucose concentration and body temperature. However, as is evident from the way sicknesses cause fever, the body temperature also fluctuates due to factors other than blood glucose concentration. While methods have been proposed to determine blood glucose concentration by temperature measurement without blood sampling, they could hardly be considered sufficiently accurate.

It is an object of the invention to provide a method and apparatus for determining blood glucose concentration with high accuracy based on temperature data regarding a test subject without blood sampling.

Blood sugar is delivered to the cells throughout the human body via blood vessel systems, particularly the capillary blood vessels. In the human body, complex metabolic pathways exist. Glucose oxidation is a reaction in which, fundamentally, blood sugar reacts with oxygen to produce water, carbon dioxide, and energy. Oxygen herein refers to the oxygen delivered to the cells via blood. The volume of oxygen supply is determined by the blood hemoglobin concentration, the hemoglobin oxygen saturation, and the volume of blood flow. On the other hand, the heat produced in the body by glucose oxidation is dissipated from the body by convection, heat radiation, conduction, and so on. On the assumption that the body temperature is determined by the balance between the amount of energy produced in the body by glucose burning, namely heat production, and heat dissipation such as mentioned above, the inventors set up the following model:

(1) The amount of heat production and the amount of heat dissipation are considered equal.
(2) The amount of heat production is a function of the blood glucose concentration and the volume of oxygen supply.
(3) The volume of oxygen supply is determined by the blood hemoglobin concentration, the blood hemoglobin oxygen saturation, and the volume of blood flow in the capillary blood vessels.
(4) The amount of heat dissipation is mainly determined by heat convection and heat radiation.

According to this model, we achieved the present invention after realizing that blood sugar levels can be accurately determined on the basis of the results of measuring the temperature of the body surface and parameters relating to the blood oxygen concentration and the blood flow volume. The parameters can be measured, e.g., from a part of the human body, such as the fingertip. The parameters relating to convection and radiation can be determined by measuring the temperature on the fingertip. The parameters relating to the blood hemoglobin concentration and the blood hemoglobin oxygen saturation can be determined by spectroscopically measuring blood hemoglobin and then finding the ratio between hemoglobin bound with oxygen and hemoglobin not bound with oxygen. With regard to the parameters relating to the blood hemoglobin concentration and blood hemoglobin oxygen saturation, instead of actually performing measurements, constants that are stored in advance may be used without adversely affecting the measurement accuracy. The parameter relating to the volume of blood flow can be determined by measuring the amount of heat transfer from the skin.

In one example, the invention provides a blood sugar level measuring apparatus comprising:

a heat amount measurement portion for measuring a plurality of temperatures deriving from a body surface and obtaining information used for calculating the amount of heat transferred by convection and the amount of heat transferred by radiation, both related to the dissipation of heat from said body surface;
an oxygen amount measuring portion for obtaining information about blood oxygen amount;
a memory portion for storing relationships between parameters corresponding to said plurality of temperatures and blood oxygen amount and blood sugar levels;
a calculating portion which converts a plurality of measurement values inputted from said heat amount measuring portion and said oxygen amount measurement portion into said parameters, and which calculates a blood sugar level by applying said parameters to said relationship stored in said memory portion; and
a display portion for displaying the blood sugar level calculated by said calculating portion,
wherein:
said oxygen amount measurement portion includes a blood flow volume measurement portion for obtaining information about blood flow volume, and an optical measurement portion for obtaining hemoglobin concentration and hemoglobin oxygen saturation in blood, wherein said blood flow volume measurement portion includes:
a body-surface contact portion;
a first temperature detector disposed adjacent to said body-surface contact portion;
a second temperature detector for detecting the temperature at a position spaced apart from said body-contact portion;
a heat-conducting member connecting said body-surface contact portion and said second temperature detector, wherein:
said heat-conducting member comprises a cylindrical main body and a plate-like support plate disposed in an internal cavity of said main body in the longitudinal direction of said main body, wherein said first temperature detector is fixed to an end portion of said support plate and disposed in said internal cavity of said main body in such a manner that it does not come into contact with said main body, and wherein said second temperature detector is fixed on an external surface of said main body.

In another example, the invention provides a blood sugar level measuring apparatus comprising:

an ambient temperature measuring portion for measuring ambient temperature;

a body-surface contact portion to be brought into contact with a body surface;

a first temperature detector disposed adjacent to said body-surface contact portion;

a radiation heat detector for measuring radiation heat from said body surface;

a heat-conducting member disposed adjacent to said body-surface contact portion;

a second temperature detector disposed adjacent to said heat-conducting member at a position spaced apart from said body-surface contact portion, for detecting the temperature at the position spaced apart from said body-surface contact portion;

a light source for irradiating said body-surface contact portion with light of at least two different wavelengths;

a photodetector for detecting reflected light produced as said light is reflected on said body surface;

a calculating portion including a conversion portion for converting the outputs of said first temperature detector, said second temperature detector, said ambient temperature measuring portion, said radiation heat detector, and said photodetector into parameters, and a processing portion in which relationships between said parameters and blood sugar levels are stored in advance, said processing portion calculating a blood sugar level by applying said parameters to said relationships; and

a display portion for displaying the result outputted from said calculation portion, wherein said heat-conducting member comprises a cylindrical main body and a plate-like support plate disposed in an internal cavity of said main body in the longitudinal direction of said main body, wherein said first temperature detector is fixed to an end portion of said support plate and disposed in said internal cavity of said main body in such a manner that it does not come into contact with said main body, and wherein said second temperature detector is fixed on an external surface of said main body.

In yet another example, the invention provides a blood sugar level measuring apparatus comprising:

an ambient temperature measuring portion for measuring ambient temperature;

a body-surface contact portion with which a body surface is brought into contact;

a first temperature detector disposed adjacent to said body-surface contact portion;

a radiation heat detector for measuring radiation heat from said body surface;

a heat-conducting member disposed adjacent to said body-surface contact portion;

a second temperature detector disposed adjacent to said heat-conducting member at a position spaced apart from said body-surface contact portion, for detecting the temperature at the position spaced apart from said body-surface contact portion;

a memory portion in which information regarding blood hemoglobin concentration and hemoglobin oxygen saturation is stored;

a calculation portion including a conversion portion for converting the outputs of said first temperature detector, said second temperature detector, said ambient temperature measuring portion, and said radiation heat detector, into a plurality of parameters, and a processing portion in which relationships between said parameters and blood sugar levels are stored, said calculation portion calculating a blood sugar level by applying said parameters to said relationships; and

a display portion for displaying the result outputted from said calculation portion, wherein said heat-conducting member comprises a cylindrical main body and a plate-like support plate disposed in an internal cavity of said main body in the longitudinal direction of said main body, wherein said first temperature detector is fixed to an end portion of said support plate and disposed in said internal cavity of said main body in such a manner that it does not come into contact with said main body, and wherein said second temperature detector is fixed on an external surface of said main body. When displaying the result outputted from the calculation portion, a calculated blood sugar level may be displayed, or a particular score corresponding to the blood sugar level may be displayed.

In accordance with the invention, blood sugar levels can be determined through noninvasive measurement with similar levels of accuracy to the conventional invasive methods.

BRIEF DESCRIPTION OF THE DRAWINGS

Fig. 1 shows a model of heat transfer from a body surface to a block.

Fig. 2 shows temporal changes in the measurement values of temperatures $T_1$ and $T_2$.

Fig. 3 shows an example of measurement of a temporal change in temperature $T_3$.

Fig. 4 shows the relationship between measurement values obtained by various sensors and parameters derived therefrom.

Fig. 5 shows a top plan view of a non-invasive blood sugar level measuring apparatus according to the invention.

Fig. 6 shows an operation procedure for the apparatus.

Fig. 7 shows the details of a measurement portion.

Fig. 8 shows heat transfer in an ideal state.

Fig. 9 shows heat transfer in a state different from the ideal state.

Fig. 10 shows a structure of the block.

Fig. 11 is a drawing for the explanation of an effect of the block.

Fig. 12 is a drawing for the explanation of an effect of the block.

Fig. 13 is a drawing for the explanation of an effect of the block.

Fig. 14 is a conceptual chart illustrating the flow of data processing in the apparatus.

Fig. 15 is a chart plotting the glucose concentration values calculated by the invention and the glucose concentration values measured by the enzyme electrode method.

Fig. 16 shows the details of another example of the measurement portion.

Fig. 17 is a conceptual chart showing data storage locations in the apparatus.

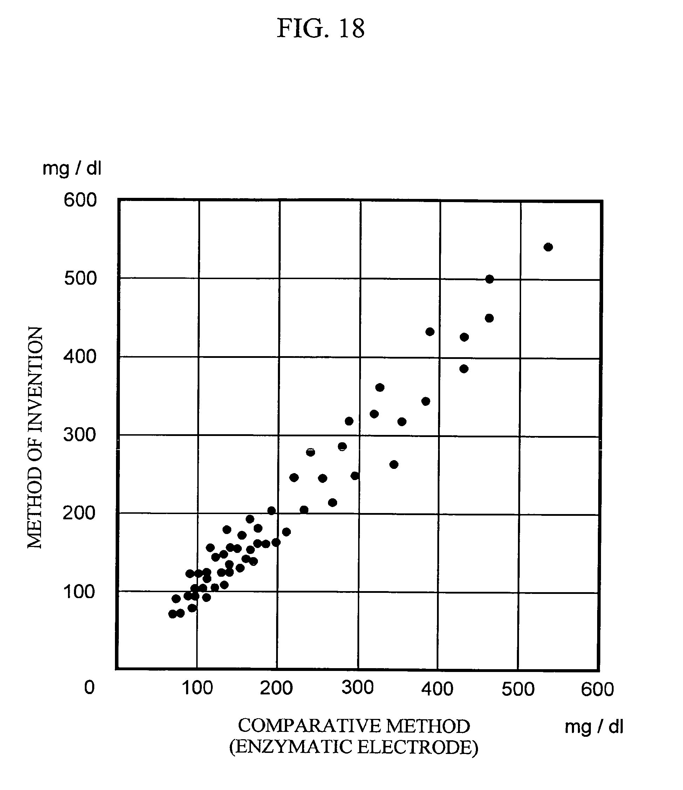

Fig. 18 is a chart plotting the glucose concentration values calculated by the invention and the glucose concentration values measured by the enzyme electrode method.

Fig. 19 is a drawing for the explanation of the dependency of the amount of heat exchanged between a copper foil pattern formed on a temperature sensor support plate and a block main body on distance Ld.

DETAILED DESCRIPTION OF THE PREFERRED EMBODIMENTS

The invention will now be described by way of preferred embodiments thereof with reference made to the drawings, in which similar functional portions are designated by similar reference numerals for ease of understanding.

Initially, the above-mentioned model will be described in more specific terms. Regarding the amount of heat dissipation, convective heat transfer, which is one of the main causes of heat dissipation, is related to temperature difference between the ambient (room) temperature and the body-surface temperature. The amount of heat dissipation due to radiation, which is another main cause of dissipation, is proportional to the fourth power of the body-surface temperature according to the Stefan-Boltzmann law. Thus, it can be seen that the amount of heat dissipation from the human body is related to the room temperature and the body-surface temperature. On the other hand, the amount of oxygen supply, which is a major factor related to the amount of heat production, is expressed as the product of hemoglobin concentration, hemoglobin oxygen saturation, and blood flow volume.

The hemoglobin concentration can be measured from the absorbance at the wavelength at which the molar absorbance coefficient of the oxyhemoglobin is equal to that of the reduced (deoxy-) hemoglobin (equal-absorbance wavelength). The hemoglobin oxygen saturation can be measured by measuring the absorbance at the equal-absorbance wavelength and the absorbance at at least one different wavelength at which the ratio between the molar absorbance coefficient of the oxyhemoglobin and that of the reduced (deoxy-) hemoglobin is known, and then solving simultaneous equations. Namely, the hemoglobin concentration and hemoglobin oxygen saturation can be obtained by conducting the measurement of absorbance at at least two wavelengths.

The rest is the blood flow volume, which can be measured by various methods. One example will be described below.

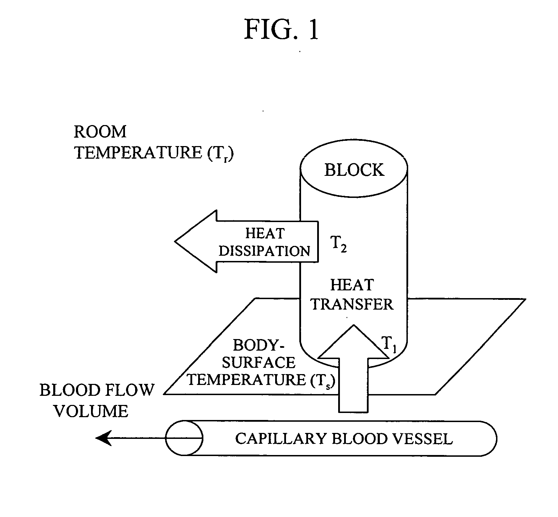

Fig. 1 shows a model for the description of the transfer of heat from the body surface to a solid block having a certain heat capacity when the block is brought into contact with the body surface for a certain time and then separated. The block is made of resin such as plastic or vinyl chloride. In the illustrated example, attention will be focused on the temporal variation of the temperature $T_1$ of a portion of the block that is brought into contact with the body surface, and the temporal variation of the temperature $T_2$ at a point on the block spaced apart from the body surface. The blood flow volume can be estimated by monitoring mainly the temporal variation of the temperature $T_2$ (at the spatially separated point on the block). The details will follow.

Before the block comes into contact with the body surface, the temperatures $T_1$ and $T_2$ at the two points of the block are equal to the room temperature $T_r$. When a body-surface temperature $T_s$ is higher than the room temperature $T_r$ as the block comes into contact with the body surface, the temperature $T_1$ swiftly rises due to the transfer of heat from the skin, and it approaches the body-surface temperature $T_s$. On the other hand, the temperature $T_2$ is lowered from the temperature $T_1$ as the heat conducted through the block is dissipated from the block surface, and it rises more gradually. The temporal variation of the temperatures $T_1$ and $T_2$ depends on the amount of heat transferred from the body surface to the block, which in turn depends on the blood flow volume in the capillary blood vessels under the skin. If the capillary blood vessels are regarded as a heat exchanger, the coefficient of transfer of heat from the capillary blood vessels to the surrounding cell tissues is given as a function of the blood flow volume. Thus, by measuring the amount of heat transfer from the body surface to the block by monitoring the temporal variation of the temperatures $T_1$ and $T_2$,

the amount of heat transferred from the capillary blood vessels to the cell tissues can be estimated. Based on this estimation, the blood flow volume can then be estimated. Thus, by tracking the temperature change in $T_1$ and $T_2$ over time and thereby measuring the amount of heat transferred from the body surface to the block, the heat transfer amount from the capillary blood vessels to the cell tissue can be estimated, which in turn allows for the estimation of the blood flow volume.

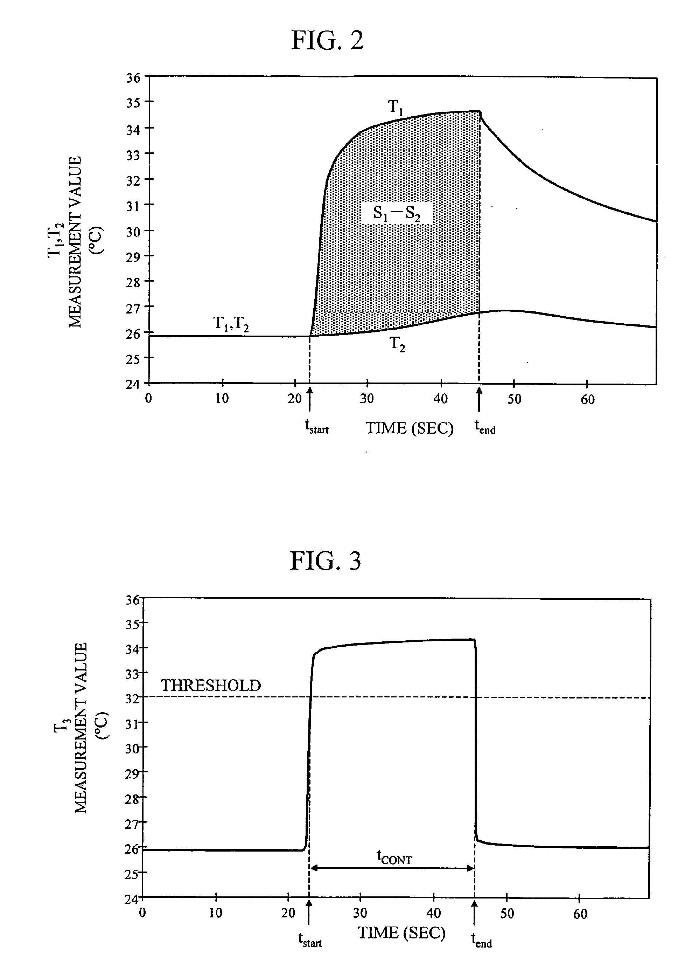

Fig. 2 shows the temporal variation of the measured values of the temperature $T_1$ at the portion of the block in contact with the body surface and the temperature $T_2$ at the position on the block spaced apart from the body-surface contact position. As the block comes into contact with the body surface, the $T_1$ measured value swiftly rises, and it gradually drops as the block is brought out of contact.

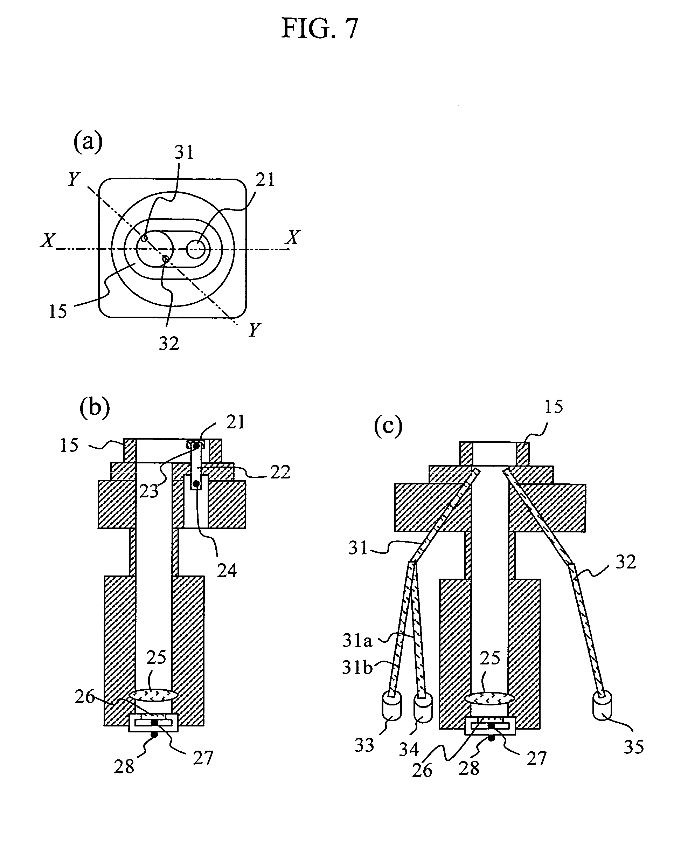

Fig. 3 shows the temporal variation of the value of the temperature $T_3$ measured by a radiation-temperature detector. As the detector detects the temperature $T_3$ that is due to radiation from the body surface, it is more sensitive to temperature changes than other sensors. Because radiation heat propagates as an electromagnetic wave, it can transmit temperature changes instantaneously. Thus, by locating the radiation-temperature detector near where the block contacts the body surface so as to detect radiated heat from the body surface, as shown in Fig. 7 (which will be described later), the time of start of contact $t_{start}$ and the time of end of contact $t_{end}$ between the block and the body surface can be detected from changes in the temperature $T_3$. For example, a temperature threshold value is set as shown in Fig. 3. The contact start time $t_{start}$ is when the temperature threshold value is exceeded. The contact end time $t_{end}$ is when the temperature $T_3$ drops below the threshold. The temperature threshold is set at 32˚C, for example.

Then, the $T_1$ measured value between $t_{start}$ and tend is approximated by an S curve, such as a logistic curve. A logistic curve is expressed by the following equation:

$$T = \frac{b}{1 + c \times \exp(-a \times t)} + d$$

where T is temperature, and t is time.

The measured value can be approximated by determining coefficients a, b, c, and d using the non-linear least-squares method. For the resultant approximate expression, T is integrated between time $t_{start}$ and time tend to obtain a value $S_1$.

Similarly, an integrated value $S_2$ is calculated from the $T_2$ measured value. The smaller $(S_1 - S_2)$ is, the larger the amount of transfer of heat is from the finger surface to the position of $T_2$. $(S_1 - S_2)$ becomes larger with increasing finger-surface contact time $T_{CONT}$ (=$t_{end}$ - $t_{start}$). Thus, $a_5/(t_{CONT} \times (S_1 - S_2))$ is designated as a parameter $X_5$ indicating the volume of blood flow, using $a_5$ as a proportionality coefficient.

It will be seen from the above discussion that the measured amounts necessary for the determination of blood glucose concentration by the above-described model are the room temperature (ambient temperature), body surface temperature, temperature changes in the block brought into contact with the body surface, the temperature due to radiation from the body surface, and absorbance at at least two wavelengths.

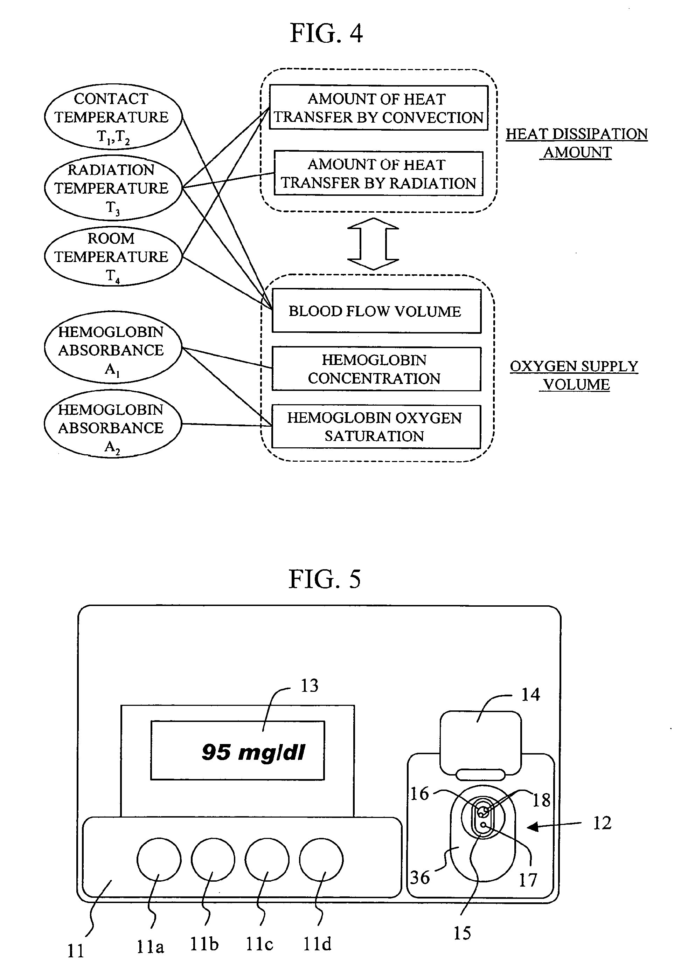

Fig. 4 shows the relationships between the measured values provided by various sensors and the parameters derived therefrom. A block is brought into contact with the body surface, and chronological changes in two kinds of temperatures $T_1$ and $T_2$ are measured by two temperature sensors provided at two locations of the block. Separately, radiation temperature $T_3$ on the body surface and room temperature $T_4$ are measured. Absorbance $A_1$ and $A_2$ are measured at at least two wavelengths related to the absorption of hemoglobin. The temperatures $T_1$, $T_2$, $T_3$, and $T_4$ provide parameters related to the volume of blood flow. The temperature $T_3$ provides a parameter related to the amount of heat transferred by radiation. The temperatures $T_3$ and $T_4$ provide parameters related to the amount of heat transferred by convection. The absorbance $A_1$ provides a parameter related to the hemoglobin concentration, and absorbance $A_1$ and $A_2$ provide parameters related to the hemoglobin oxygen saturation.

Hereafter, an example of an apparatus for non-invasively measuring blood sugar levels according to the principle of the invention will be described.

Fig. 5 shows a top plan view of a non-invasive blood sugar level measuring apparatus according to the invention. While in this example the skin on the ball of the fingertip is used as the body surface, other parts of the body surface may be used.

On the top surface of the apparatus are provided an operating portion 11, a measuring portion 12 where the finger to be measured is to be placed, and a display portion 13 for displaying measurement results, the state of the apparatus, measured values, for example. The operating portion 11 includes four push buttons 11a to 11d for operating the apparatus. The measuring portion 12 has a cover 14 which, when opened (as shown), reveals a finger rest portion 15 with an oval periphery in a finger rest guide 36. The finger rest portion 15 accommodates an opening end 16 of a radiation-temperature sensor portion, a contact-temperature sensor portion 17, and an optical sensor portion 18.

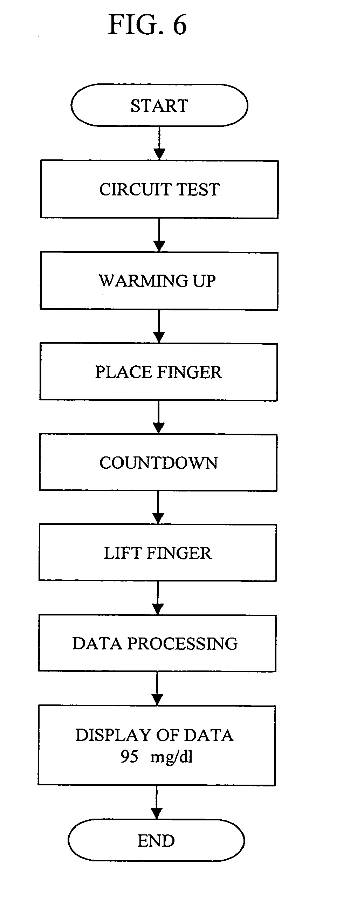

Fig. 6 shows a procedure for operating the apparatus. As a power button on the operating portion is pressed to turn on the apparatus, an indication "WARMING UP" is displayed on the LCD and the electronic circuits in the apparatus are warmed up. At the same time, a check program is activated to automatically check the electronic circuits. After the warm-up phase is finished, an indication "PLACE FINGER" appears on the LCD. As the user places his or her finger on the finger rest portion, a countdown is displayed on the LCD. When the countdown is over, an indication "LIFT FINGER" appears on the LCD. As the user puts his or her finger away, the LCD indicates "PROCESSING DATA." Thereafter, the display shows a blood sugar level, which is then stored in an IC card together with the date and time. After the user reads the displayed blood sugar level, he or she pushes a particular button on the operating portion. About one minute later, the apparatus displays a message "PLACE FINGER" on the LCD, thus indicating that the apparatus is ready for the next cycle of measurement.

Fig. 7 shows the measuring portion in detail. In Fig. 7, (a) is a top plan view, (b) is a cross section taken along line X-X of (a), and (c) is a cross section taken along line Y-Y of (a).

First, the process of measuring temperatures by the non-invasive blood sugar level measuring apparatus according to the invention will be described. In a portion of the measuring portion with which the examined portion (ball of the finger) is to come into contact, a thin plate 21 of a highly heat-conductive material, such as gold, is placed. A bar-shaped heat-conductive member 22, which is made of a material with a heat conductivity lower than that of the plate 21, such as polyvinylchloride, is thermally connected to the plate 21 and extends into the apparatus. The temperature sensors include a thermistor 23 that is an adjacent-temperature detector with respect to the examined portion for measuring the temperature of the plate 21, and a thermistor 24 that is an indirect-temperature detector with respect to the examined portion for measuring the temperature of a portion of the heat-conducting member which is spaced apart from the plate 21 by a certain distance. An infrared lens 25 is disposed inside the apparatus at such a position that the examined portion (ball of the finger) placed on the finger rest portion 15 can be seen through the lens. Below the infrared lens 25 is disposed a pyroelectric detector 27 via an infrared radiation-transmitting window 26. Another thermistor 28 is disposed in close proximity to the pyroelectric detector 27.

Thus, the temperature sensor portion of the measuring portion includes four temperature sensors, and they measure four kinds of temperatures as follows:

(1) Temperature on the finger surface (thermistor 23): $T_1$
(2) Temperature of the heat-conducting member (thermistor 24): $T_2$
(3) Temperature of radiation from the finger (pyroelectric detector 27): $T_3$
(4) Room temperature (thermistor 28): $T_4$

As described above, the blood sugar level measuring apparatus of the invention utilizes the heat-conducting member in the form of a block (with a length L (m), a diameter R (m), and heat characteristics, such as heat conductivity $\lambda$ (J/s·m·K), or heat capacity U (J/K: specific heat capacity cv (J/K·kg) x block density $\rho$ (kg/m³) x block volume V (m³)) for estimating the magnitude of the blood flow volume, as shown in Figs. 1 and 7. This is so that the amount of heat transmitted from the heat source (such as the finger surface) to the heat-conducting member ("block") 22 can be determined from a temperature distribution on the block by bringing the block into contact with the heat source. As shown in Fig. 7, on the block 22, there are disposed the temperature detector (thermistor) 23 for measuring the temperature of the heat source, and the temperature detector (thermistor) 24 for measuring the temperature distribution on the block 22. In order to reduce the contact thermal resistance between the block 22 and the heat source, a plate 21 made of a substance with a high heat conductivity, such as gold, is provided where the block 22 and the heat source are brought into contact with each other. Furthermore, the metal plate 21 and the block 22 are thermally actively connected with each other via a low thermal resistance.

When calculating the temperature distribution and the heat amount accurately using the block 22 with the above-described structure, the temperature sensors per se and the method of mounting them must be carefully considered. Specifically, if the temperature distribution and the heat amount were to be accurately calculated, it would be required that the mass of the temperature sensors be such that it can be regarded as substantially zero, so that they would not affect the flow of heat from the heat source to the block. However, this is an ideal state and is actually impossible to realize. Therefore, how a measurement environment that is as close to the ideal state as possible can be created using the existing components with realistic dimensions and mass largely determines the measurement accuracy. To be more specific, it is necessary to control the flow of heat to and from the temperature sensors so as to get as close to the ideal state as possible.

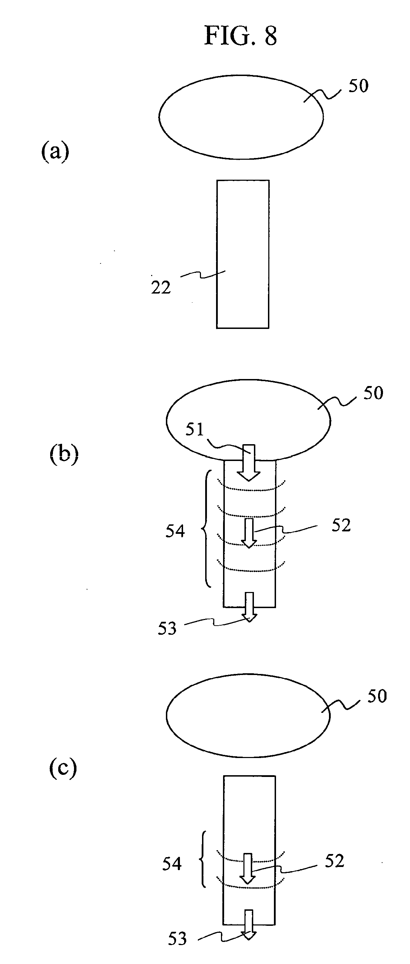

Fig. 8 shows the flow of heat from the heat source to the block in the ideal state. Fig. 8(a) shows a state prior to the heat source 50 making contact with the block 22. The block 22 is in a state of thermal equilibrium with the surrounding environment, and there is no temperature distribution in the block 22. Fig. 8(b) shows a state in which the heat source 50 is in contact with the block 22, so that there is a temperature distribution 54 in the block 22 due to the heat conducted from the heat source 50. In this state, the heat 51 that passes through a contact plane between the heat source 50 and the block 22 flows inside the block 22 (52), and is then emitted to the outside of the block 22 (53). Although Fig. 8(b) depicts the heat being emitted only from the bottom portion of the block, the emission of heat takes place throughout the surface of the block depending on the temperature distribution thereon. When the heat source 50 and the block 22 are separated from each other, as shown in Fig. 8(c), the heat stored inside the block 22 upon contact with the heat source 50 is emitted to the outside, so that the temperature distribution decreases and eventually reaches the state shown in Fig. 8(a). In the blood sugar level measuring apparatus of the invention, the above-described thermal cycle is repeated for each measurement. In a measurement using the above-described block, the temperature distribution 54 and the surface temperature of the heat source 50 as they are present in the states shown in Fig.8(b) and (c) are measured.

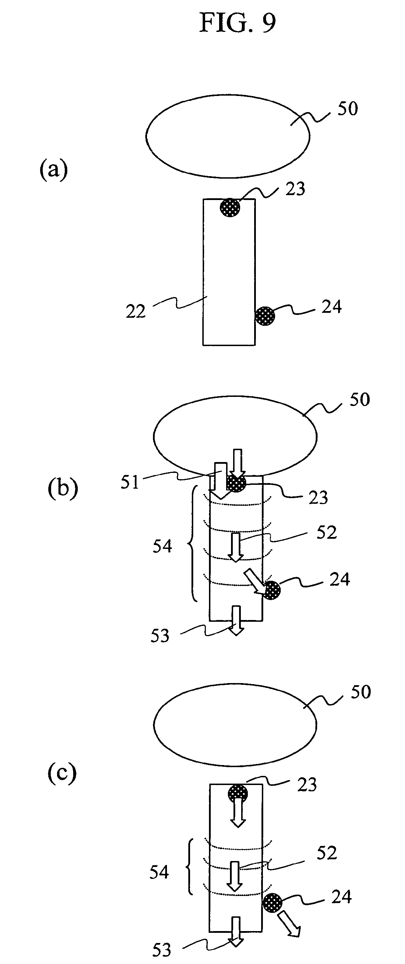

Fig. 9 shows the flow of heat from the heat source to the block in a case where the measurement environment is created using realizable components. The difference between this and the ideal state lies in the heat capacity of the temperature sensor T1 (specific heat capacity, volume, and density), and the state of thermal coupling between the temperature sensor and the block, for example. Hereafter, problems associated with the aforementioned difference will be discussed.

Fig. 9(a) shows the state prior to the heat source 50 coming into contact with the block 22. The block 22 is in a state of thermal equilibrium with the susrrounding environment, and so there is no temperature distribution in the block 22. Similarly, the sensors 23 and 24 are in a state of thermal equilibrium with the block 22. The state shown in Fig. 9(b) is such that the heat source 50 is in contact with the block 22, so that there is a temperature distribution 54 inside the block 22 due to heat conduction from the heat source 50. Heat 51 that passes through a contact plane between the heat source 50 and the block 22 flows inside the block 22 (52), and is then emitted to the outside of the block 22 (53). At the same time, the heat is also supplied to the sensors 23 and 24, each of which detects the temperature at the location where each is installed. Further, as the heat source 50 and the block 22 are separated from each other, as shown in Fig. 9(c), the heat that has been stored inside the block 22 by the contact between the heat source and the block is emitted to the outside of the block. As a result, the heat distribution decreases and eventually reaches the state of Fig. 8(a). At the same time, the sensors 23 and 24 also emit heat, which creates the problem of the influence of the heat emitted from the sensor 23 via the block 22 on the temperature distribution. Generally, sensors and block materials have different physical properties, so that different amounts of heat are stored in them. In addition, a large contact thermal resistance is conceivably produced in the path of emission of the stored heat, resulting in different emission times and leading to the development of a temperature distribution, after separation from the heat source, that is different from the ideal state. As a result, measurement accuracy drops and so does the accuracy of the blood sugar levels calculated by the blood sugar level measuring apparatus of the invention.

In order to reduce the error created by the aforementioned reasons and to improve the accuracy of the blood sugar level measuring apparatus of the invention, the structure of the block (including the manner of installation of the temperature sensors) in accordance with the invention is designed as follows.

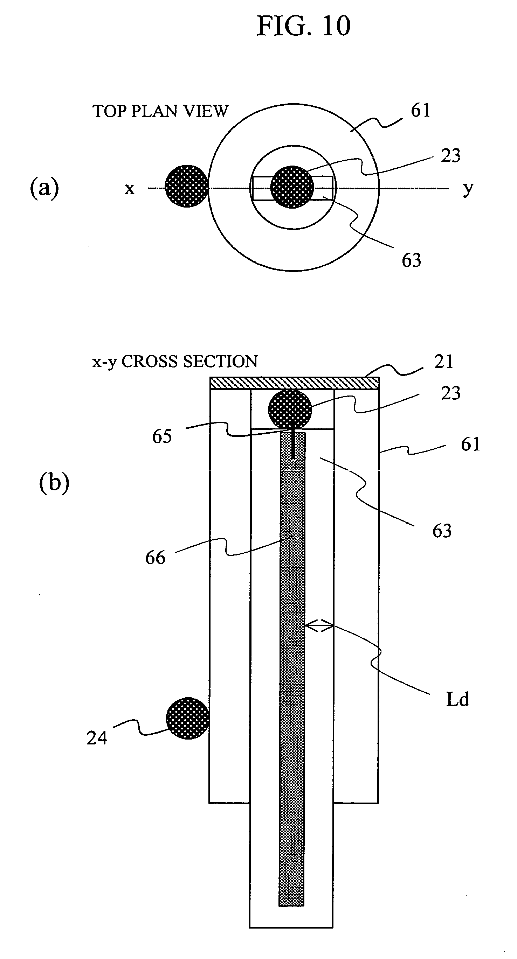

Fig. 10 shows an example of the structure of the block in accordance with the invention. Fig. 10(a) shows the structure of the block in a direction perpendicular to the plane of contact with the heat source (heat-source contact plane). Although not shown, a metal plate is covered on the heat-source contact plane. In the illustrated example, a block main body 61 is cylindrical in shape and includes a concentric internal cavity. Inside the cavity, there is disposed the temperature sensor 23 for measuring the temperature on the plane of contact with the heat source. More specifically, the temperature sensor 23 is fixedly installed on a temperature sensor support plate 63 with glue. The temperature sensor support plate 63 and the cylinder internal walls of the block main body 61 are in contact with one another with a mechanical friction coefficient such that the temperature sensor support plate 63 is fixed to the block main body 61. It should be noted, however, that this method of bringing the temperature sensor support plate 63 into contact with the cylinder internal walls of the block main body 61 and thereby fixing the former to the latter involving a mechanical friction coefficient is merely an example. Alternatively, glue may be used to make sure that the fastening is reliable. In this case, however, care must be taken to ensure that the adhesion takes place via a part and not the whole of the mechanical contact portion, so as to prevent an increase in heat conductivity.

The contact between the temperature sensor support plate 63 and the cylinder internal walls of the block main body 61 is via line contact at the four corners of the temperature sensor support plate 63. Examples of the material of the temperature support plate 63 include paper phenol and glass epoxy resin. A material with a low heat conductivity is preferable.

Fig. 10(b) shows a cross section taken along line xy indicated in Fig. 10(a). Fig. 10(b) also shows the metal plate 21, which Fig. 10(a) omitted. The metal plate 21 is disposed in a cross-sectional plane of the cylinder such that it covers the heat-source contact plane, as shown. The temperature sensor 23 is positioned to be in contact with the metal plate 21. In the present embodiment, the temperature sensors are thermistors. Because thermistors utilize a change in resistance value depending on temperature, they require electric signal line connections. For this purpose, on the temperature sensor support plate 63, there is formed a signal pattern 66 made of copper foil, for example, for transmitting signals for the detection of resistance value change in the temperature sensor 23. A signal line 65 from the temperature sensor 23 is connected to a signal pattern 66. It should be obvious that the copper-foil pattern is merely an example, and that the signal pattern may also be formed by other material, such as aluminum or gold. With reference to Fig. 10 (b), numeral 24 designates a temperature sensor provided for measuring the temperature of the block main body 61. The temperature sensor 24 is preferably in contact with the block main body 61 via a small thermal resistance. For example, the sensor may be fixed with glue in such a manner as to increase the contact area. Alternatively, a part of the temperature sensor 24 may be embedded in the block main body 61, with or without glue.

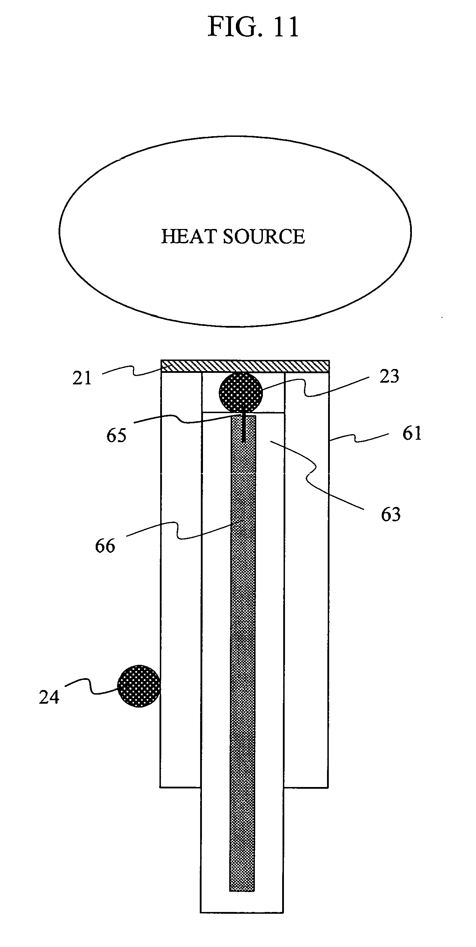

With reference to Figs. 11, 12, and 13, the effect of incorporating the above-described structure of the block into a measurement cycle including the aforementioned contact with and separation from the heat source.

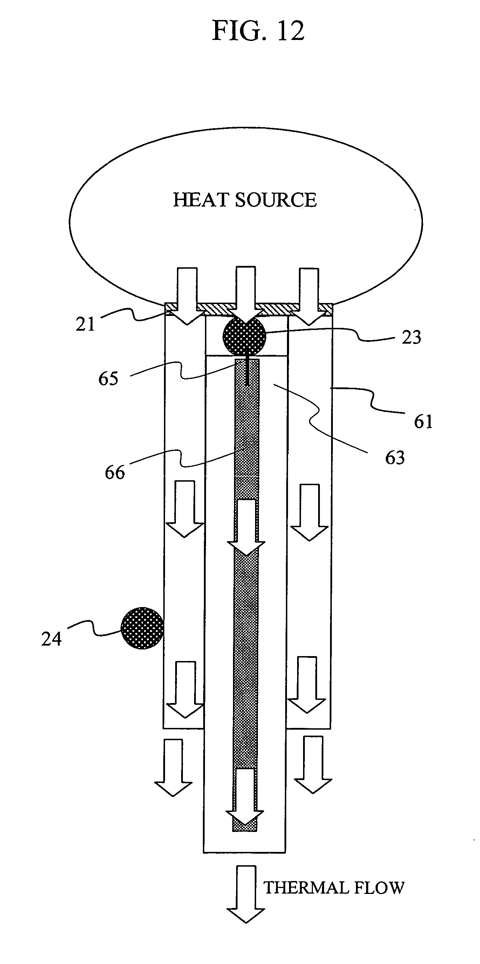

Fig. 11 shows a state prior to making contact with the heat source, where the block is in a state of thermal equilibrium with the environment. Fig. 12 shows a state after the block is brought into contact with the heat source. The heat from the heat source flows via the metal plate into the sensor for measuring the temperature at the plane of contact between the cylinder, namely the block main body, and the heat source. The purpose of this measurement is to determine the amount of heat that has flowed into the cylindrical block based on the temperature distribution produced thereon. Thus, the temperature distribution must be prevented from being influenced by heat other than the heat that has flowed into the cylinder. The "heat other than the heat that has flowed into the cylinder" herein refers to the heat that has flowed into the sensor 23 for measuring the temperature on the heat-source contact plane. In the present embodiment, the temperature sensor is disposed on the temperature sensor support plate, on which the copper foil pattern 66 with good heat conductivity is formed. Therefore, the heat that has flowed into the sensor 23 for measuring the temperature on the heat-source contact plane is emitted outside the cylinder via the copper foil pattern 66, as well as being stored in the temperature sensor 23. In this case, none of the heat that is conducted by the copper foil pattern on the temperature sensor support plate flows into the cylinder, nor from the cylinder to the temperature sensor support plate due to the poor heat conductivity of the material of the temperature sensor support plate 63 and to the mechanical contact with thermal resistance between the temperature sensor support plate 66 and the cylinder internal walls.

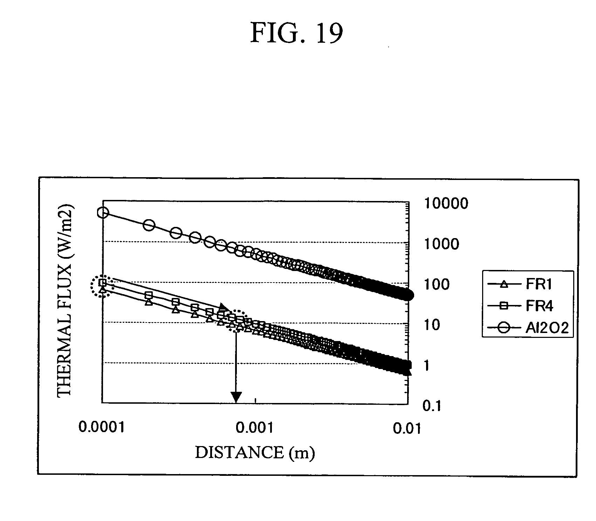

The heat conductivity, which is determined by a distance Ld between the internal walls of the block main body 61 and the signal pattern 66, and by the material of the temperature sensor support plate, is an important parameter that determines the amount of heat exchanged between the copper foil pattern on the temperature sensor support plate and the block main body. Fig. 19 is a drawing for the explanation of the dependency of the amount of heat exchanged between the copper foil pattern on the temperature sensor support plate and the block main body on the distance Ld between the internal walls of the block main body 61 and the signal pattern 66, with reference to three different kinds of materials of a substrate with a common thickness (A (m)), in the case where there is a temperature difference of 0.05°C between the copper foil pattern on the temperature sensor support plate and the block main body. The materials of the substrate are: (1) paper phenol (FR1, heat conductivity: 0.13 W/m.K); (2) glass epoxy resin (FR4, heat conductivity: 0.19. W/m.K); and (3) alumina ($Al_2O_2$) ceramic (heat conductivity: 10 W/m.K). The amount of heat conducted is inversely proportional to the thermal resistance (the product of thermal conductivity and distance). For example, by selecting a distance for each of the aforementioned materials such that the conducted heat amount is substantially one tenth that when the distance Ld is approximately 0, a highly accurate measurement can be made that is not subject to the influence of the conducted heat. When (1) paper phenol (FR1) or (2) glass epoxy resin (FR4) is selected as the material of the substrate, unwanted thermal flux can be prevented by providing a maximum of 0.9 mm of interval.

As described above, in the case of substrate materials with a low heat conductivity, such as paper phenol and glass epoxy resin, unwanted thermal flux can be blocked (1/10) when the gap between the conductor pattern edge and the block internal walls is 1 mm or less. However, in the case of substrate materials with a high heat conductivity, such as alumina, it takes a gap of approximately a dozen millimeters in order to block unwanted thermal flux. Thus, in order to realize physical dimensions to be applied to the heat measurement portion of the invention and to perform a highly accurate measurement by blocking unwanted thermal flux, it is necessary that the heat conductivity of the substrate material be not more than 1 W/m·K, in consideration of the heat conductivity of the material, typically paper phenol or glass epoxy resin, and the heat resistance that exists in the contact portion between the substrate and the block internal walls.

Although in the present example the temperature difference that is produced within the physical dimensions (several millimeters) of the heat measurement portion of the invention is set to be 0.05°C, expected temperature differences are not more than 0.1 °C or so. By reducing the thermal flux between these temperature differences to approximately 1/10, the maximum value of the error in the temperature that is to be originally measured can be reduced to approximately 1/10 of 0.1°C, or 0.01°C. In this way, a highly accurate measurement can be made without being affected by the heat that is conducted.

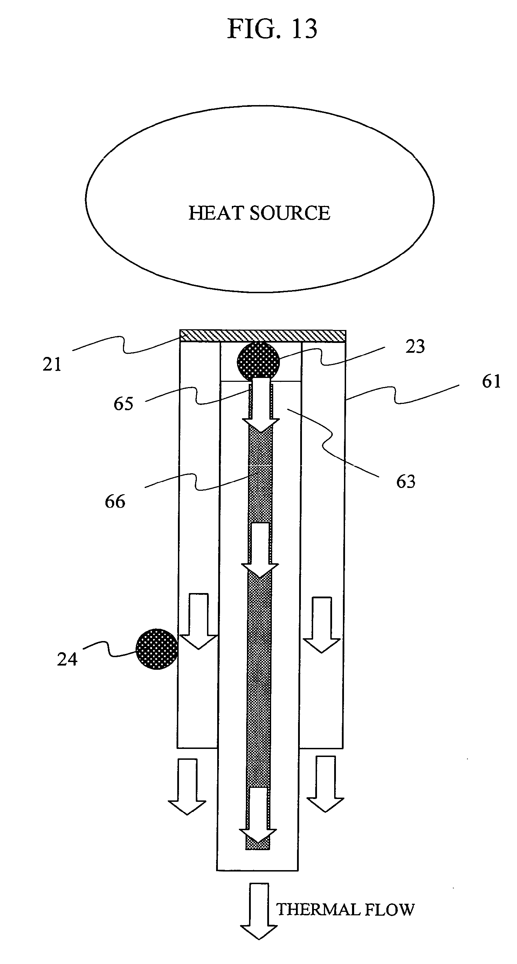

Fig. 13 shows a state after the block has been separated from the heat source, when the heat that has been stored in the block main body or the temperature sensor is emitted. In this case, too, the heat stored in the block main body is emitted via the block main body, and the heat stored in the temperature sensor is emitted via the copper foil pattern with good heat conductivity formed on the temperature sensor support plate. Thus, the heat is emitted separately and does not become mixed.

As described above, the invention enables a block to be formed that is close to the ideal state, whereby measurement accuracy can be improved.

The optical sensor portion 18 is described hereafter. The optical sensor portion 18 measures the hemoglobin concentration and the hemoglobin oxygen saturation necessary for the determination of the oxygen supply volume. In order to measure the hemoglobin concentration and the hemoglobin oxygen saturation, it is necessary to measure absorbance at at least two wavelengths. Fig. 7(c) shows an exemplary configuration for carrying out a two-wavelength measurement using two light sources 33 and 34 and a single detector 35.

The ends of two optical fibers 31 and 32 are located in the optical sensor portion 18. The optical fiber 31 is for optical irradiation, while the optical fiber 32 is for receiving light. As shown in Fig. 7(c), the optical fiber 31 connects to branch optical fibers 31a and 31b, and the ends thereof are provided with light-emitting diodes 33 and 34 of two wavelengths. The receiving optical fiber 32 is provided at the end thereof with a photodiode 35. The light-emitting diode 33 emits light with a wavelength of 810 nm, while the light-emitting diode 34 emits light with a wavelength of 950 nm. The wavelength 810 nm is the equal-absorbance wavelength at which the molar absorbance coefficient of the oxyhemoglobin is equal to that of the reduced (deoxy-) hemoglobin. The wavelength 950 nm is the wavelength at which the difference between the molar absorbance coefficient of the oxyhemoglobin and that of the reduced hemoglobin is large.

The two light-emitting diodes 33 and 34 emit light in a time-sharing manner. The finger of an examined subject is irradiated with the light emitted by the light-emitting diodes 33 and 34 via the light-irradiating optical fiber 31. The light shone on the finger is reflected by the skin of the finger and is then incident on the light-receiving optical fiber 32, via which the light is detected by the photodiode 35. When the light with which the finger is irradiated is reflected by the skin of the finger, part of the light penetrates into the tissue through the skin and is absorbed by the hemoglobin in the blood that flows in capillary blood vessels. The measurement data provided by the photodiode 35 is reflectance R. Absorbance can be approximately calculated from log(1/R). Irradiation is conducted with light of wavelengths 810 nm and 950 nm, R is measured for each, and then log(1/R) is obtained, thereby measuring absorbance $A_1$ for wavelength 810 nm and absorbance $A_2$ for wavelength 950 nm.

When the reduced hemoglobin concentration is [Hb] and the oxyhemoglobin concentration is [HbO$_2$], absorbance $A_1$ and absorbance $A_2$ are expressed by the following equations:

$$A_1 = a \times ([Hb] \times A_{Hb}(810nm) + [HbO_2] \times A_{HbO_2}(810nm))$$
$$= a \times ([Hb] + [HbO_2]) \times A_{HbO_2}(810nm)$$

$$A_2 = a \times ([Hb] \times A_{Hb}(950nm) + [HbO_2] \times A_{HbO_2}(950nm))$$
$$= a \times ([Hb] + [HbO_2]) \times ((1 - \frac{[HbO_2]}{[Hb] + [HbO_2]}) \times A_{Hb}(950nm) + \frac{[HbO_2]}{[Hb] + [HbO_2]} \times A_{HbO_2}(950nm))$$

where $A_{Hb}$(810 nm) and $A_{Hb}$(950 nm), and $A_{Hb02}$(810 nm) and $A_{Hb02}$(950 nm) are the molar absorbance coefficients of the reduced hemoglobin and the oxyhemoglobin, respectively, and are known at the respective wavelengths. The term a is a proportionality coefficient. From the above equations, the blood hemoglobin concentration ([Hb]+[HbO$_2$])$_T$ inside tissue and the blood hemoglobin oxygen saturation ([HbO$_2$]/([Hb]+[HbO$_2$]))$_T$ are determined as follows:

$$[Hb] + [HbO_2] \approx \frac{A_1}{a \times A_{HbO_2}(810nm)}$$

$$\frac{[HbO_2]}{[Hb] + [HbO_2]} = \frac{A_2 \times A_{HbO_2}(810nm) - A_1 \times A_{Hb}(950nm))}{A_1 \times (A_{HbO_2}(950nm) - A_{Hb}(950nm))}$$

While the above example involved the measurement of the hemoglobin concentration and hemoglobin oxygen saturation based on the measurement of absorbance at two wavelengths, it is also possible to reduce the influence of interfering components and increase measurement accuracy by measuring absorbance at three or more wavelengths.

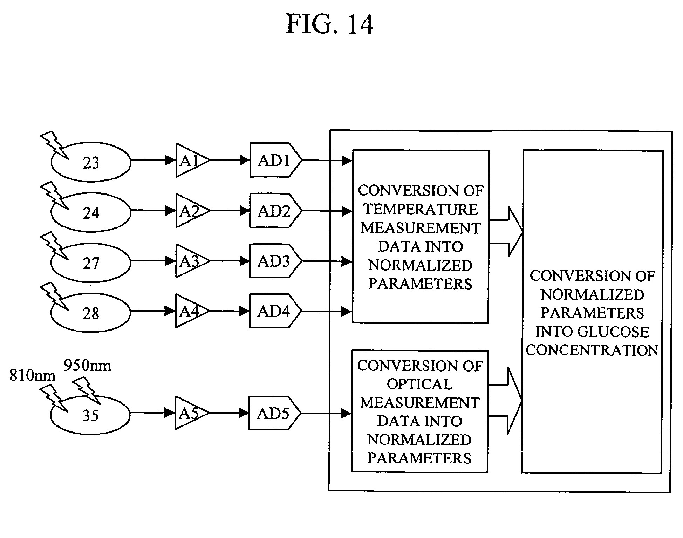

Fig. 14 is a conceptual chart showing the flow of data processing in the apparatus. The apparatus according to the present example is equipped with five sensors, namely a thermistor 23, a thermistor 24, a pyroelectric detector 27, a thermistor 28, and a photodiode 35. The photodiode 35 measures absorbance at wavelengths 810 nm and 950 nm. Thus, the apparatus is supplied with six kinds of measurement values.

The five kinds of analog signals are supplied via individual amplifiers A1 to A5 to analog/digital converters AD1 to AD5, where they are converted into digital signals. Based on the digitally converted values, parameters $x_i$ (i=1, 2, 3, 4, 5) are calculated. The following are specific descriptions of $x_i$ (where $a_1$ to $a_5$ are proportionality coefficients):

Parameter proportional to heat radiation

$$x_1 = a_1 \times (T_3)^4$$

Parameter proportional to heat convection

$$x_2 = a_2 \times (T_4 - T_3)$$

Parameter proportional to hemoglobin concentration

$$x_3 = a_3 \left( \frac{A_1}{a \times A_{HbO_2}(810nm)} \right)$$

Parameter proportional to hemoglobin saturation

$$x_4 = a_4 \times \left( \frac{A_2 \times A_{HbO_2}(810nm) - A_1 \times A_{Hb}(950nm))}{A_1 \times (A_{HbO_2}(950nm) - A_{Hb}(950nm))} \right)$$

Parameter proportional to oxygen supply volume

$$x_5 = a_5 \times \left( \frac{1}{t_{CONT} \times (S_1 - S_2)} \right)$$

Then, normalized parameters are calculated from mean values and standard deviations of parameters $x_i$ obtained from actual data on large numbers of able-bodied people and diabetic patients. A normalized parameter $X_i$ (where i=1, 2, 3, 4, 5) is calculated from each parameter $x_i$ according to the following equation:

$$X_i = \frac{x_i - \bar{x}_i}{SD(x_i)}$$

where

$x_i$: parameter

$\overline{x_i}$: mean value of the parameter

$SD(x_i)$: standard deviation of the parameter

Calculations are conducted to convert the above five normalized parameters into a glucose concentration to be eventually displayed. A program necessary for the calculations is stored in the ROM built inside the microprocessor in the apparatus. A memory area necessary for the calculations is ensured in a RAM similarly built inside the apparatus. The result of the calculations is displayed on the LCD portion.

The ROM stores, as a constituent element of the program necessary for the computations, a function for determining glucose concentration C in particular. This function is defined as follows. C is expressed by a below-indicated equation (1), where $a_i$ (i=0, 1, 2, 3, 4, 5) is determined from a plurality of pieces of measurement data in advance according to the following procedure:

(1) A multiple regression equation is created that indicates the relationship between the normalized parameter and the glucose concentration C.

(2) Normalized equations (simultaneous equations) relating to the normalized parameter are obtained from an equation obtained by the least-squares method.

(3) Values of coefficient $a_i$ (i=0, 1, 2, 3, 4, 5) are determined from the normalized equation and then substituted into the multiple regression equation.

Initially, the regression equation (1) indicating the relationship between the glucose concentration C and the normalized parameters $X_1$, $X_2$, $X_3$, $X_4$ and $X_5$ is formulated.

$$C = f(X_1, X_2, X_3, X_4, X_5)$$
$$= a_0 + a_1 X_1 + a_2 X_2 + a_3 X_3 + a_4 X_4 + a_5 X_5 \quad \ldots\ldots(1)$$

Then, the least-squares method is employed to obtain a multiple regression equation that would minimize the error with respect to a measured value $C_i$ of glucose concentration according to an enzyme electrode method. When the sum of the squares of the residual is D, D is expressed by the following equation (2):

$$D = \sum_{i=1}^{n} d_i^{\,2}$$
$$= \sum_{i=1}^{n} (C_i - f(X_{i1}, X_{i2}, X_{i3}, X_{i4}, X_{i5}))^2$$
$$= \sum_{i=1}^{n} \{C_i - (a_0 + a_1 X_{i1} + a_2 X_{i2} + a_3 X_{i3} + a_4 X_{i4} + a_5 X_{i5})\}^2 \quad \ldots\ldots(2)$$

The sum of the squares of the residual D becomes minimum when partial differentiation of equation (2) with respect to $a_0$, $a_2$, ..., $a_5$ gives zero. Thus, we have the following equations:

$$\frac{\partial D}{\partial a_0} = -2\sum_{i=1}^{n}\{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_1} = -2\sum_{i=1}^{n} X_{i1}\{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_2} = -2\sum_{i=1}^{n} X_{i2}\{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_3} = -2\sum_{i=1}^{n} X_{i3}\{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_4} = -2\sum_{i=1}^{n} X_{i4}\{C_i - (a_0 + a_1 X_{i1} + a_2 X_{i2} + a_3 X_{i3} + a_4 X_{i4} + a_5 X_{i5})\} = 0$$

$$\frac{\partial D}{\partial a_5} = -2\sum_{i=1}^{n} X_{i5}\{C_i - (a_0 + a_1 X_{i1} + a_2 X_{i2} + a_3 X_{i3} + a_4 X_{i4} + a_5 X_{i5})\} = 0 \quad ......(3)$$

When the mean values of C and $X_1$ to $X_5$ are $C_{mean}$ and $X_{1mean}$ to $X_{5mean}$, respectively, since $X_{imean}=0$ (i=1 to 5), equation (1) yields equation (4) thus:

$$a_0 = C_{mean} - a_1 X_{1mean} - a_2 X_{2mean} - a_3 X_{3mean} - a_4 X_{4mean} - a_5 X_{5mean}$$
$$= C_{mean} \qquad ......(4)$$

The variation and covariation between the normalized parameters are expressed by equation (5). Covariation between the normalized parameter $X_i$ (i=1 to 5) and C is expressed by equation (6).

$$S_{ij} = \sum_{k=1}^{n}(X_{ki} - X_{imean})(X_{kj} - X_{jmean}) = \sum_{k=1}^{n} X_{ki} X_{kj} \quad (i, j = 1,2,..5) \quad ......(5)$$

$$S_{iC} = \sum_{k=1}^{n}(X_{ki} - X_{imean})(C_k - C_{mean}) = \sum_{k=1}^{n} X_{ki}(C_k - C_{mean}) \quad (i = 1,2,..5) \quad ......(6)$$

Substituting equations (4), (5), and (6) into equation (3) and rearranging yields simultaneous equations (normalized equations) (7). Solving equations (7) yields $a_1$ to $a_5$.

$$a_1 S_{11} + a_2 S_{12} + a_3 S_{13} + a_4 S_{14} + a_5 S_{15} = S_{1C}$$
$$a_1 S_{21} + a_2 S_{22} + a_3 S_{23} + a_4 S_{24} + a_5 S_{25} = S_{2C}$$
$$a_1 S_{31} + a_2 S_{32} + a_3 S_{33} + a_4 S_{34} + a_5 S_{35} = S_{3C}$$
$$a_1 S_{41} + a_2 S_{42} + a_3 S_{43} + a_4 S_{44} + a_5 S_{45} = S_{4C}$$
$$a_1 S_{51} + a_2 S_{52} + a_3 S_{53} + a_4 S_{54} + a_5 S_{55} = S_{5C} \quad ......(7)$$

Constant term $a_0$ is obtained by means of equation (4). The thus obtained $a_i$ (i=0, 1, 2, 3, 4, 5) is stored in ROM at the time of manufacture of the apparatus. In actual measurement using the apparatus, the normalized parameters $X_1$ to $X_5$ obtained from the measured values are substituted into regression equation (1) to calculate the glucose concentration C.

Hereafter, an example of the process of calculating the glucose concentration will be described. The coefficients in equation (1) are determined in advance based on a large quantity of data obtained from able-bodied persons and diabetic patients. The ROM in the microprocessor stores the following formula for the calculation of glucose concentration:

$$C = 99.4 + 18.3 \times X_1 - 20.2 \times X_2 - 23.7 \times X_3 - 22.0 \times X_4 - 25.9 \times X_5$$

$X_1$ to $X_5$ are the results of normalization of parameters $x_1$ to $x_5$. Assuming the distribution of the parameters is normal, 95% of the normalized parameters take on values between -2 and +2.

In an example of measured values for an able-bodied person, substituting normalized parameters $X_1$=-0.06, $X_2$=+0.04 and $X_3$=+0.05, $X_4$=-0.12 and $X_5$=+0.10 in the above equation yields C=96 mg/dL. In an example of measured values for a diabetic patient, substituting normalized parameters $X_1$=+1.15, $X_2$=-1.02, $X_3$=-0.83, $X_4$=-0.91 and $X_5$=-1.24 in the equation yields C=213 mg/dL.

Hereafter, the results of measurement by the conventional enzymatic electrode method and those by the embodiment of the invention will be described. In the enzymatic electrode method, a blood sample is reacted with a reagent and the amount of resultant electrons is measured to determine the blood sugar level. When the glucose concentration was 89 mg/dL according to the enzymatic electrode method in an example of measured values for an able-bodied person, substituting normalized parameters $X_1$=-0.06, $X_2$=+0.04, $X_3$=+0.05, $X_4$=-0.12 and $X_5$=+0.10 obtained by measurement at the same time according to the inventive method into the above equation yield C=96 mg/dL. Further, when the glucose concentration was 238 mg/dL according to the enzymatic electrode method in an example of measurement values for a diabetic patient, substituting $X_1$=+1.15, $X_2$=-1.02, $X_3$=-0.83, $X_4$=-0.91 and $X_5$=-1.24 obtained by measurement at the same time according to the inventive method yields C=213 mg/dL. From the above results, it has been confirmed that the glucose concentration can be accurately determined using the method of the invention.

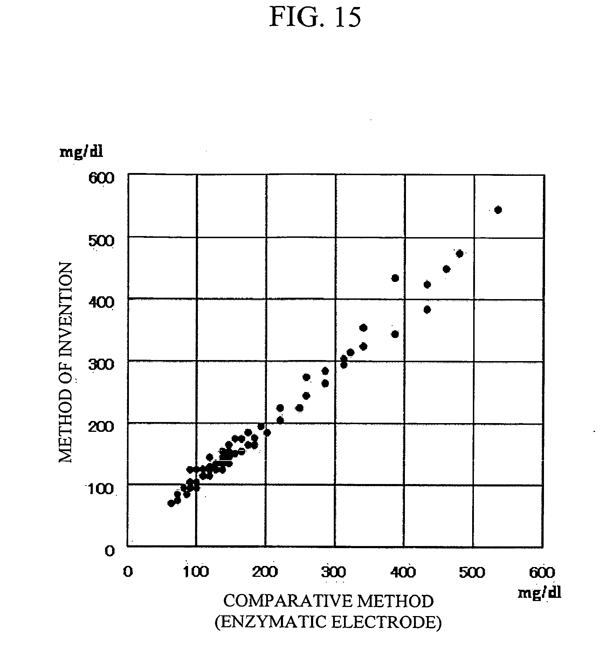

Fig. 15 shows a chart plotting on the vertical axis the values of glucose concentration calculated by the inventive method and on the horizontal axis the values of glucose concentration measured by the enzymatic electrode method, based on a plurality of patients. A good correlation is obtained by measuring the oxygen supply volume and blood flow volume according to the invention (correlation coefficient = 0.9324).

In the above-described embodiment, the parameters relating to blood hemoglobin concentration and blood hemoglobin oxygen saturation have been obtained by spectroscopically measuring the hemoglobin in blood. However, the hemoglobin concentration is stable in persons without such symptoms as anemia, bleeding or erythrocytosis. The hemoglobin concentration is normally in the range between 13 to 18 g/dL for males and between 12 to 17 g/dL for females, and the range of variation of hemoglobin concentration from the normal values is 5 to 6%. Further, the weight of the term relating to the blood flow volume in the aforementioned formula for calculating blood sugar level is smaller than other terms. Therefore, the hemoglobin concentration can be treated as a constant without greatly lowering the measurement accuracy. Similarly, the hemoglobin oxygen saturation is stable between 97 to 98% if the person is undergoing aerial respiration at atmospheric pressure, at rest and in a relaxed state. Thus the hemoglobin concentration and the hemoglobin oxygen saturation can be treated as constants, and the oxygen supply volume can be determined from the product of the hemoglobin concentration constant, the hemoglobin oxygen saturation constant and the blood flow volume.

By treating the hemoglobin concentration and hemoglobin oxygen saturation as constants, the sensor arrangement for measuring blood sugar level can be simplified by removing the optical sensors, for example. Further, by eliminating the time necessary for optical measurement and the processing thereof, the procedure for blood sugar level measurement can be accomplished in less time.

Because the hemoglobin oxygen saturation takes on a stable value when at rest, in particular, by treating the hemoglobin concentration and hemoglobin oxygen saturation as constants, the measurement accuracy for blood sugar level measurement when at rest can be increased, and the procedure for blood sugar level measurement can be accomplished in less time. By "when at rest" herein is meant the state in which the test subject has been either sitting on a chair or lying and thus moving little for approximately five minutes.

Hereafter, an embodiment will be described in which the blood hemoglobin concentration and blood hemoglobin oxygen saturation are treated as constants. This embodiment is similar to the above-described embodiment except that the blood hemoglobin concentration and blood hemoglobin oxygen saturation are treated as constants, and therefore the following description mainly concerns the differences from the earlier embodiment.

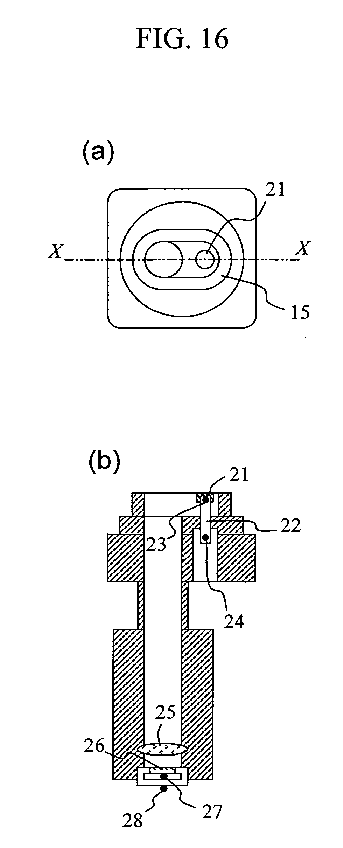

In the present embodiment, the hemoglobin concentration and hemoglobin oxygen saturation shown in Fig. 4 are not measured but treated as constants. Therefore, as shown in Fig. 16, the measurement portion of the present embodiment has the structure of the measurement portion of the earlier embodiment shown in Fig. 7 from which the light sources 33 and 34, photodiode 35 and optical fibers 31 and 32 have been removed. Parameters used in the present embodiment are parameter $x_1$ proportional to heat radiation, parameter $x_2$ related to heat convection, and parameter $x_3$ proportional to the oxygen supply volume (hereafter, parameter proportional to oxygen supply volume will be indicated as $x_3$). From these parameters, normalized parameters are calculated in the manner described above, and a glucose concentration is calculated based on the three normalized parameters $X_i$ (i=1, 2, 3). During data processing, the step "CONVERSION OF OPTICAL MEASUREMENT DATA INTO NORMALIZED PARAMETERS" (see Fig. 14), which is necessary in the previous embodiment, can be eliminated.

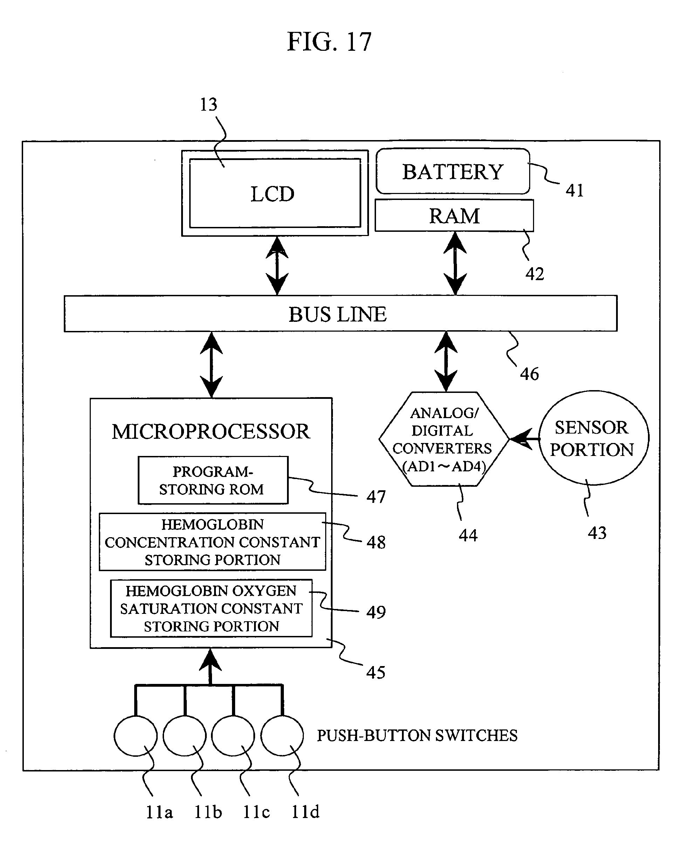

Fig. 17 shows a functional block diagram of the apparatus according to the embodiment. The apparatus runs on battery 41. Signals measured by sensor portion 43 including a temperature sensor are fed to analog/digital converters 44 (AD1 to AD4) provided for individual signals where they are converted into digital signals. Analog/digital converters AD1 to AD4, LCD 13 and RAM 42 are peripheral circuits to microprocessor 45. They are accessed by the microprocessor 45 via bus line 46. The push buttons 11a to 11d are connected to microprocessor 45. The microprocessor 45 includes the ROM for storing software. By pressing the buttons 11a to 11d, external instructions can be entered into microprocessor 45.

The ROM 47 included in the microprocessor 45 stores a program necessary for computations, i.e., it has the function of an arithmetic unit. The microprocessor 45 further includes a hemoglobin concentration constant storage portion 48 for storing hemoglobin concentration constants, and a hemoglobin oxygen saturation constant storage portion 49 for storing hemoglobin oxygen saturation constants. After the measurement of the finger is finished, the computing program calls up optimum constants from the hemoglobin concentration storage portion 48 and hemoglobin oxygen saturation constant storage portion 49 and perform calculations. A memory area necessary for computations is ensured in the RAM 42 similarly incorporated into the apparatus. The result of computations is displayed on the LCD portion.

The ROM stores, as a constituent element of the program necessary for the computations, a function for determining glucose concentration C in particular. The function is defined as follows. C is expressed by a below-indicated equation (8), where $a_i$ (i=0, 1, 2, 3) is determined from a plurality of pieces of measurement data in advance according to the following procedure:

(1) A multiple regression equation is created that indicates the relationship between the normalized parameter and the glucose concentration C.
(2) Normalized equations (simultaneous equations) relating to the normalized parameter are obtained from an equation obtained by the least-squares method.
(3) Values of coefficient $a_i$ (i=0, 1, 2, 3) are determined from the normalized equation and then substituted into the multiple regression equation.

Initially, the regression equation (8) indicating the relationship between the glucose concentration C and the normalized parameters $X_1$, $X_2$ and $X_3$ is formulated.

$$C = f(X_1, X_2, X_3)$$
$$= a_0 + a_1 X_1 + a_2 X_2 + a_3 X_3 \quad \ldots\ldots(8)$$

Then, the least-squares method is employed to obtain a multiple regression equation that would minimize the error with respect to a measured value $C_i$ of glucose concentration according to an enzyme electrode method. When the sum of squares of the residual is D, D is expressed by the following equation (9):

$$D = \sum_{i=1}^{n} d_i^2$$
$$= \sum_{i=1}^{n} (C_i - f(X_{i1}, X_{i2}, X_{i3}))^2$$
$$= \sum_{i=1}^{n} \{C_i - (a_0 + a_1 X_{i1} + a_2 X_{i2} + a_3 X_{i3})\}^2 \quad \ldots\ldots(9)$$

The sum of squares of the residual D becomes minimum when partial differentiation of equation (9) with respect

to $a_0$ to $a_3$ gives zero. Thus, we have the following equations:

$$\frac{\partial D}{\partial a_0} = -2\sum_{i=1}^{n}\{C_i - (a_0 + a_1 X_{i1} + a_2 X_{i2} + a_3 X_{i3})\} = 0$$

$$\frac{\partial D}{\partial a_1} = -2\sum_{i=1}^{n} X_{i1}\{C_i - (a_0 + a_1 X_{i1} + a_2 X_{i2} + a_3 X_{i3})\} = 0$$

$$\frac{\partial D}{\partial a_2} = -2\sum_{i=1}^{n} X_{i2}\{C_i - (a_0 + a_1 X_{i1} + a_2 X_{i2} + a_3 X_{i3})\} = 0$$

$$\frac{\partial D}{\partial a_3} = -2\sum_{i=1}^{n} X_{i3}\{C_i - (a_0 + a_1 X_{i1} + a_2 X_{i2} + a_3 X_{i3})\} = 0 \quad ......(10)$$

When the mean values of C and $X_1$ to $X_3$ are $C_{mean}$ and $X_{1mean}$ to $X_{3mean}$, respectively, since $X_{imean}=0$ (i=1 to 3), equation (8) yields equation (11) thus:

$$a_0 = C_{mean} - a_1 X_{1mean} - a_2 X_{2mean} - a_3 X_{3mean}$$
$$= C_{mean} \qquad\qquad ......(11)$$

The variation and covariation between the normalized parameters are expressed by equation (12). Covariation between the normalized parameter $X_i$ (i=1 to 3) and C is expressed by equation (13).

$$S_{ij} = \sum_{k=1}^{n}(X_{ki} - X_{imean})(X_{kj} - X_{jmean}) = \sum_{k=1}^{n} X_{ki} X_{kj} \quad (i, j = 1,2,3) \quad ......(12)$$

$$S_{iC} = \sum_{k=1}^{n}(X_{ki} - X_{imean})(C_k - C_{mean}) = \sum_{k=1}^{n} X_{ki}(C_k - C_{mean}) \quad (i = 1,2,3) \quad ......(13)$$

Substituting equations (11), (12), and (13) into equation (10) and rearranging yields simultaneous equations (normalized equations) (14). Solving equations (14) yields $a_1$ to $a_3$.

$$a_1 S_{11} + a_2 S_{12} + a_3 S_{13} = S_{1C}$$
$$a_1 S_{21} + a_2 S_{22} + a_3 S_{23} = S_{2C}$$
$$a_1 S_{31} + a_2 S_{32} + a_3 S_{33} = S_{3C} \quad ......(14)$$

Constant term $a_0$ is obtained by means of equation (11). The thus obtained $a_i$ (i=0, 1, 2, 3) is stored in ROM at the time of manufacture of the apparatus. In actual measurement using the apparatus, the normalized parameters $X_1$ to $X_3$ obtained from the measured values are substituted into regression equation (8) to calculate the glucose concentration C.

Hereafter, an example of the process of calculating the glucose concentration will be described. The coefficients in equation (8) are determined in advance based on a large quantity of data obtained from able-bodied persons and diabetic patients. The ROM in the microprocessor stores the following formula for the calculation of glucose concentration:

$$C = 101.7 + 25.8 \times X_1 - 23.2 \times X_2 - 12.9 \times X_3$$

$X_1$ to $X_3$ are the results of normalization of parameters $x_1$ to $x_3$. Assuming the distribution of the parameters is normal, 95% of the normalized parameters take on values between -2 and +2.

In an example of measured values for an able-bodied person, substituting normalized parameters $X_1$=-0.06, $X_2$=+0.04 and $X_3$=+0.10 in the above equation yields C=101 mg/dL. In an example of measured values for a diabetic patient, substituting normalized parameters $X_1$=+1.35, $X_2$=-1.22 and $X_3$=-1.24 in the equation yields C=181 mg/dL. In the above equation, the hemoglobin concentration and hemoglobin oxygen saturation are rendered into constants of 15 g/dL and 97%, respectively.

Hereafter, the results of measurement by the conventional enzymatic electrode method and those by the embodiment of the invention will be described. In the enzymatic electrode method, a blood sample is reacted with a reagent and the amount of resultant electrons is measured to determine glucose concentration. When the glucose concentration was 93 mg/dL according to the enzymatic electrode method in an example of measured values for an able-bodied person, substituting normalized parameters $X_1$=-0.06, $X_2$=+0.04 and $X_3$=+0.10 obtained by measurement at the same time according to the inventive method into the above equation yielded C=101 mg/dL. Further, when the glucose concentration was 208 mg/dL according to the enzymatic electrode method in an example of measurement values for a diabetic patient, substituting $X_1$=+1.35, $X_2$=-1.22 and $X_3$=-1.24 obtained by measurement at the same time according to the inventive method yielded C=181 mg/dL. Although the calculation results indicate an error of about 13%, this level of accuracy is considered sufficient because normally errors between 15% and 20% are considered acceptable in blood sugar level measuring apparatuses in general. Thus, it has been confirmed that the method of the invention can allow glucose concentrations to be determined with high accuracy.

Fig. 21 shows a chart plotting on the vertical axis the values of glucose concentration calculated by the inventive method and on the horizontal axis the values of glucose concentration measured by the enzymatic electrode method, based on measurement values obtained from a plurality of patients. A good correlation is obtained by measuring according to the invention (correlation coefficient = 0.8932).

## Claims

1. A blood sugar level measuring apparatus comprising:

   a heat amount measurement portion (21-28) for measuring a plurality of temperatures deriving from a body surface and obtaining information used for calculating the amount of heat transferred by convection and the amount of heat transferred by radiation, both related to the dissipation of heat from said body surface;
   an oxygen amount measurement portion (21-28, 31-35) for obtaining information about the blood oxygen amount;
   a memory portion (47, 48, 49) for storing relationships between parameters corresponding to said plurality of temperatures and said blood oxygen amount, and blood sugar levels;
   a calculating portion (45) which converts a plurality of measurement values inputted from said heat amount measuring portion and said oxygen amount measurement portion into said parameters, and which computes a blood sugar level by applying said parameters to said relationships stored in said storage portion; and
   a display portion (13) for displaying the result calculated by said calculating portion,
   wherein:
   said oxygen amount measurement portion includes a blood flow volume measurement portion for obtaining information about the blood flow volume, and an optical measurement portion (31-35) for obtaining the hemoglobin concentration and hemoglobin oxygen saturation in blood, wherein said blood flow volume measurement portion includes:
   a body-surface contact portion (15, 21);
   a first temperature detector (23) disposed adjacent to said body-surface contact portion;
   a second temperature detector (24) for detecting the temperature at a position spaced apart from said body-contact potion; and
   a heat-conducting member (22) connecting said body-surface contact portion with said second temperature detector, wherein:
   said heat-conducting member comprises a cylindrical main body (61) and a plate-like support plate (63) disposed in an internal cavity of said main body in the longitudinal direction of said main body, wherein said first temperature detector (23) is fixed to an end portion of said support plate and disposed in said internal cavity of said main body in such a manner that it does not come into contact with said main body, and wherein said second temperature detector (24) is fixed on an external surface of said main body.

2. A blood sugar level measuring apparatus comprising:

an ambient temperature measurement portion (28) for measuring ambient temperature;
a body-surface contact portion (15, 21) with which a body surface is brought into contact;
a first temperature detector (23) disposed adjacent to said body-surface contact portion;
a radiation heat detector (25-27) for measuring radiation heat from said body surface;
a heat-conducting member (22) disposed adjacent to said body-surface contact portion;
a second temperature detector (24) disposed adjacent to said heat-conducting member at a position spaced apart from said body-surface contact portion for detecting the temperature at the position spaced apart from said body-surface contact portion;
a light source (31, 33, 34) for irradiating said body-surface contact portion with light of at least two different wavelengths;
a photodetector (32, 35) for detecting reflected light produced as said light is reflected on said body surface;
a calculating portion (45) including a conversion portion for converting the outputs of said first temperature detector, said second temperature detector, said ambient temperature measuring portion, said radiation heat detector, and said photodetector into parameters, and a processing portion in which relationships between said parameters and blood sugar levels are stored in advance, said processing portion calculating a blood sugar level by applying said parameters to said relationships; and
a display portion (13) for displaying the result outputted from said calculation portion, wherein:
said heat-conducting member (22) comprises a cylindrical main body (61) and a plate-like support plate (63) disposed in an internal cavity of said main body in the longitudinal direction of said main body, wherein said first temperature detector (23) is fixed to an end portion of said support plate and disposed in said internal cavity of said main body in such a manner that it does not come into contact with said main body, and wherein said second temperature detector (24) is fixed on an external surface of said main body.

3. A blood sugar level measuring apparatus comprising:

an ambient temperature measuring portion (28) for measuring ambient temperature;
a body-surface contact portion (15, 21) with which a body surface is brought into contact;
a first temperature detector (23) disposed adjacent to said body-surface contact portion;
a radiation heat detector (25-27) for measuring radiation heat from said body surface;
a heat-conducting member (22) disposed adjacent to said body-surface contact portion;
a second temperature detector (24) disposed adjacent to said heat-conducting member at a position spaced apart from said body-surface contact portion for detecting the temperature at the position spaced apart from said body-surface contact portion;
a memory portion (47, 48, 49) in which information regarding blood hemoglobin concentration and hemoglobin oxygen saturation is stored;
a calculation portion (45) including a conversion portion for converting the outputs of said first temperature detector, said second temperature detector, said ambient temperature measuring portion, and said radiation heat detector, into a plurality of parameters, and a processing portion in which relationships between said parameters and blood sugar levels are stored, said calculation portion calculating a blood sugar level by applying said parameters to said relationships; and
a display portion (13) for displaying the result outputted from said calculation portion, wherein:
said heat-conducting member (22) comprises a cylindrical main body (61) and a plate-like support plate (63) disposed in an internal cavity of said main body in the longitudinal direction of said main body, wherein said first temperature detector (23) is fixed to an end portion of said support plate and disposed in said internal cavity of said main body in such a manner that it does not come into contact with said main body, and wherein said second temperature detector (24) is fixed on an external surface of said main body.

4. The blood sugar level measuring apparatus according to claim 1, 2 or 3, wherein said plate-like support plate (63) is fixed within said internal cavity of said main body (61) in a mechanically contacting manner.

5. The blood sugar level measuring apparatus according to claim 1, 2 or 3, wherein a metal foil pattern (66) is formed on said plate-like support plate (63), and wherein said first temperature detector (23) is electrically connected to said pattern.

6. The blood sugar level measuring apparatus according to claim 1, 2 or 3, wherein an end portion of said cylindrical main body (61) of said heat conducting member (22) is capped with a metal plate (21) forming said body-surface contact portion, and wherein said first temperature detector (23) is in contact with said metal plate.

7. The blood sugar level measuring apparatus according to claim 1, 2 or 3, wherein said support plate (63) has a heat conductivity of 1 W/m·K or less.

# FIG. 1

## FIG. 2

## FIG. 3

## FIG. 4

| | | HEAT DISSIPATION AMOUNT |
|---|---|---|

CONTACT TEMPERATURE $T_1, T_2$

RADIATION TEMPERATURE $T_3$

ROOM TEMPERATURE $T_4$

AMOUNT OF HEAT TRANSFER BY CONVECTION

AMOUNT OF HEAT TRANSFER BY RADIATION

HEMOGLOBIN ABSORBANCE $A_1$

HEMOGLOBIN ABSORBANCE $A_2$

BLOOD FLOW VOLUME

HEMOGLOBIN CONCENTRATION

HEMOGLOBIN OXYGEN SATURATION

OXYGEN SUPPLY VOLUME

## FIG. 5

95 mg/dl

# FIG. 6

```
        ┌─────────────┐
        │    START    │
        └─────────────┘
               │
               ▼
        ┌─────────────┐
        │ CIRCUIT TEST │
        └─────────────┘
               │
               ▼
        ┌─────────────┐
        │  WARMING UP │
        └─────────────┘
               │
               ▼
        ┌─────────────┐
        │ PLACE FINGER │
        └─────────────┘
               │
               ▼
        ┌─────────────┐
        │  COUNTDOWN  │
        └─────────────┘
               │
               ▼
        ┌─────────────┐
        │ LIFT FINGER │
        └─────────────┘
               │
               ▼
        ┌─────────────────┐
        │ DATA PROCESSING │
        └─────────────────┘
               │
               ▼
        ┌─────────────────┐
        │ DISPLAY OF DATA │
        │    95  mg/dl    │
        └─────────────────┘
               │
               ▼
        ┌─────────────┐
        │     END     │
        └─────────────┘
```

# FIG. 7

(a)

(b)

(c)

# FIG. 8

(a)

(b)

(c)

# FIG. 9

(a)

(b)

(c)

# FIG. 10

TOP PLAN VIEW

(a)

x-y CROSS SECTION

(b)

# FIG. 11

# FIG. 12

# FIG. 13

HEAT SOURCE

THERMAL FLOW

FIG. 14

# FIG. 15

# FIG. 16

(a)

(b)

# FIG. 17

# FIG. 18

## FIG. 19

**European Patent Office**

**EUROPEAN SEARCH REPORT**

Application Number

EP 05 00 3466

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | WO 01/28414 A (KAUFMANN-KIM, YUN-OAK; CHO, OK-KYUNG) 26 April 2001 (2001-04-26) * page 6, line 30 - page 20 * ----- | 1-7 | A61B5/00 |
| Y | US 5 924 996 A (CHO ET AL) 20 July 1999 (1999-07-20) * the whole document * ----- | 1-7 | |
| A | WO 03/010510 A (ARGOSE, INC; MESSERSCHMIDT, ROBERT, G; MANSFIELD, JAMES; BRAND, DEREK;) 6 February 2003 (2003-02-06) * page 4, line 14 - page 9, line 24 * ----- | 1-7 | |

**TECHNICAL FIELDS SEARCHED (Int.Cl.7)**

A61B

The present search report has been drawn up for all claims

| Place of search | Date of completion of the search | Examiner |
|---|---|---|
| Munich | 28 June 2005 | Rivera Pons, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 05 00 3466

This annex lists the patent family members relating to the patent documents cited in the above-mentioned European search report.
The members are as contained in the European Patent Office EDP file on
The European Patent Office is in no way liable for these particulars which are merely given for the purpose of information.

28-06-2005

| Patent document cited in search report | | Publication date | Patent family member(s) | | Publication date |
|---|---|---|---|---|---|
| WO 0128414 | A | 26-04-2001 | WO | 0128414 A2 | 26-04-2001 |
| US 5924996 | A | 20-07-1999 | DE | 4423663 A1 | 11-01-1996 |
| | | | CA | 2194348 A1 | 18-01-1996 |
| | | | CN | 1159151 A | 10-09-1997 |
| | | | WO | 9601075 A1 | 18-01-1996 |
| | | | EP | 0771168 A1 | 07-05-1997 |
| | | | JP | 10503944 T | 14-04-1998 |
| | | | KR | 271095 B1 | 01-12-2000 |
| WO 03010510 | A | 06-02-2003 | WO | 03010510 A2 | 06-02-2003 |

EPO FORM P0459

For more details about this annex : see Official Journal of the European Patent Office, No. 12/82